# EUROPEAN PATENT APPLICATION

(11) **EP 2 641 610 A2**
(43) Date of publication of application: **25.09.2013**
(21) Application number: 11839104.4
(22) Date of filing: 11.11.2011
(51) Int. Cl.: A61K 38/16, A61K 38/17, A61P 35/00

(54) **ANTICANCER COMPOSITION CONTAINING GKN 1**

(30) Priority: 12.11.2010 KR 20100112610
(71) Applicant: Catholic University Industry Academic Cooperation Foundation, Seoul 137-701 (KR)
(72) Inventor: PARK, Won Sang, Seoul 137-040 (KR); YOON, Jung Hwan, Cheongju Chungcheongbuk-do 361-800 (KR)
(74) Representative: Bradbury, Simon Timothy Nicholas
(86) International application number: PCT/KR2011/008605
(87) International publication number: WO 2012/064146

(57) **Abstract**

The present invention relates to an anticancer composition containing an active ingredient consisting of GKN 1, and more specifically relates to an anticancer composition containing an active ingredient consisting of GKN 1 protein or an expression vector comprising the GKN 1 gene which has an outstanding anticancer activity. The GKN 1 according to the present invention is advantageous in that it can beneficially be used as a composition for anticancer therapy because it features not only outstanding activity in suppressing the occurrence of cancer cells but also outstanding activity in suppressing cancer cell metastasis during over-expression.

<110>    Industry Academic Cooperation Foundation of Catholic University

<120>    A composition comprising Gastrokine 1 for anti-cancer

<130>    OP11-1052

<150>    10-2010-0112610
<151>    2010-11-12

<160>    22

<170>    KopatentIn 2.0

<210>    1
<211>    199
<212>    PRT
<213>    Artificial Sequence

<220>
<223>    GKN 1 amino acid sequence


<400>    1
Met Leu Ala Tyr Ser Ser Val His Cys Phe Arg Glu Asp Lys Met Lys
  1               5                  10                  15

Phe Thr Ile Val Phe Ala Gly Leu Leu Gly Val Phe Leu Ala Pro Ala
             20                  25                  30

Leu Ala Asn Tyr Asn Ile Asn Val Asn Asp Asp Asn Asn Asn Ala Gly
         35                  40                  45

Ser Gly Gln Gln Ser Val Ser Val Asn Asn Glu His Asn Val Ala Asn
     50                  55                  60

Val Asp Asn Asn Asn Gly Trp Asp Ser Trp Asn Ser Ile Trp Asp Tyr
 65                  70                  75                  80

Gly Asn Gly Phe Ala Ala Thr Arg Leu Phe Gln Lys Lys Thr Cys Ile
                 85                  90                  95

Val His Lys Met Asn Lys Glu Val Met Pro Ser Ile Gln Ser Leu Asp
            100                 105                 110

Ala Leu Val Lys Glu Lys Lys Leu Gln Gly Lys Gly Pro Gly Gly Pro
        115                 120                 125

Pro Pro Lys Gly Leu Met Tyr Ser Val Asn Pro Asn Lys Val Asp Asp
    130                 135                 140

Leu Ser Lys Phe Gly Lys Asn Ile Ala Asn Met Cys Arg Gly Ile Pro
145                 150                 155                 160

Thr Tyr Met Ala Glu Glu Met Gln Glu Ala Ser Leu Phe Phe Tyr Ser
                165                 170                 175

Gly Thr Cys Tyr Thr Thr Ser Val Leu Trp Ile Val Asp Ile Ser Phe
            180                 185                 190

Cys Gly Asp Thr Val Glu Asn
        195

<210>    2
<211>    6408
<212>    DNA
<213>    Artificial Sequence

<220>
<223>    GKN 1 Polynucleotide sequence


<400>    2
ataacaccta gtttgagtca acctggttaa gtacaaatat gagaaggctt ctcattcagg      60

tccatgcttg cctactcctc tgtccactgc tttcgtgaag acaagatgaa gttcacagtg     120

agtagatttt tccttttgaa tttaccacca aatgattgga gactgtcaat attctgagat     180

ttaggaggtc tgcttcttat ggccccatca tggaaaattt gttttaaaaa aattctctct     240

tcaaacacat ggacacagag aggggaacaa cacacactag gtcctgttgg ggggtggaga     300

gtgaggggag ggaacttaga ggacaggtca ataggggcag caaaccacca tggcacacat     360

atacctatgt aacaaacctg cacgttctgc acatgtatcc ctttttttta gaagaagaaa     420

taatgaaaaa aaatcttttt tctatttata taatcatggc atttataagc atctctatag     480

agaaggataa ttgtgctgag attagacagc tgtctgagca cctcacactg acctattttt     540

aacaaaatga ctttccgcat cacctgattc cggtctccat gcagggtaag cagttcctaa     600

gccctagaaa gtgccgatca tccctcattc ttgaattcct ccttttattt accaaaattc     660

ctgagcatgt tcaggaaaga tgaaaagctt attatcaaaa taagtggctg agatagactt     720

cttgtcacat ttgttacagt aaaatgggtc tccaagaaag aaagatttgc cttgggctct     780

agcatggcca tttatttaag aaagcatctg aaacatgaag ctaccacagc atctctcctg     840

tggttccaga cagaagcctg agagtctagg aggaggtgga ccgagaaacc ctgccaaagt     900

aactagtagt gccgggtttc tcacaacacg atgcaaaggg gctagaatca gatgactatt     960

ttcatgtttc aacatactac acactggaaa acgttacggc agactctact ttataatggg    1020

gctgcaaatg taaaatgact acctagaact aggtcctctt aatagcagca aagtttaaaa    1080

gggtcagagg gagctccaga cacaggttag atttgatttc tctcctagtt ctgctgtgaa    1140

caagaggtat aagtttggcc aactcactta acccctgaag ctcagttacc ttatctgtaa    1200

aatgattgca ttgtactagg tgttctctaa aatttcttct acctctgact ttttaggaga    1260

ctaattttta actccttttt aagctattgg gagaaaaatt taattttttt tcaaaagtta    1320

ccttgaatct ctagagcagt tctcaaaact attttgtccc aggcaaagga aatgagacta    1380

ggtacccaga atgaggcacc ctgcataaag ctctgtgctc tgaaaaccaa tgtcagggac    1440

cctgtgataa ataattaaac caagtatcct gggacactgc tagtgacatc gcctctgctg    1500

atcactcttg ccagcgagac actctatact tgctttctca tcattggcat ccaaactgcc    1560

tactaatcca ttgctttgga aagttttttt taataaaaag attatttcta ttaggaagaa    1620

aacatcccat gttaaatagg aaaattaact gaaatcattt tcagatgtga tttttagcac 1680

ttatagccat ctcaaaccat agtattcatt tatactatgc tatttattgt aaaacttctt 1740

tttttttcca aggaaaataa gatagtttgc tttattttaa aacagtaact ttcttatatt 1800

ggggcactga ccaaaattca atactggtac aaatatgtta cctaggaggt caaaatatgt 1860

gccaggtgaa ttttctgaat ttctctaaag agagaatttt aaaccttata aaacaattag 1920

aaacaagtga gtgagaggtg agcatcaaca acctgtgtaa cataagccac agtacaaatt 1980

taagctgaat aaccaagcca tgtcagttat cccaaatcat ttttgttaat atttaggagg 2040

atacacatat tttcaataac ttaaaagtga atctttactc ctatctctta atactcgaag 2100

aagtataact ttcttctttt actagattta aataatccaa atatctactc aaggtaggat 2160

gctgtcatta actatagctg agtttatcca aaatagaaaa atcatgaaga tttataaagc 2220

attttaaaaa taatcattta tagcaagtcc ttgaaagctc taaataagaa aggcagttct 2280

ctactttcta ataacaccta tggtttatat tacataatat aattcaacaa aacagcattc 2340

tgaccaatga taatttatag gaaattcatt tgccaagtat atgttttatt ataaagttaa 2400

tattttgacc aatcttaaaa atttttaaac tctattctga catttccaga agtattatct 2460

tagcaaagtc atctttatga taccacttat taaactgaag agaaacaaga tggtacattc 2520

tgggttttac tttaaaaggg atttgattca ataatttgat ttatcactac ttgaaaatta 2580

cattttcttc ctcagtactg gatggcaatg agatgaaagc agctttcctg gctctcaact 2640

tcccttcttc atcaattttt ccagcgtttc ataaggccta cactaaaaat tctaaaacta 2700

tatatcacat taatataatt acttataatt aatcagcaat ttcacattat cgttaaaacc 2760

tttatggtta aaaaatgcaa ggtaagagaa gaaaaaaaca cattgaacta gaactgaaca 2820

cattggtaaa attagtgaat acttttcata agcttggata gaggaagaaa gaagacatca 2880

ttttgccatg taacaggaga ccaatgttat ttgtgatttc agattgtctt tgctggactt 2940

cttggagtct ttctagctcc tgccctagct aactatgtaa gtctcacctt ttcaagtttg 3000

ctaccaaaat gcatttgcaa ggaaatgtga tattaaatca ctctcaatct cttataaact 3060

tcagaatatc aacgtcaatg atgacaacaa caatgctgga agtgggcagc agtcagtgag 3120

tgtcaacaat gaacacaatg tggccaatgt tgacaataac aacggatggg actcctggaa 3180

ttccatctgg gattatggaa atgtaggtag tcaacgtgca attttcactt tattgtttaa 3240

aaatacgatt tctttttaac aaaaaatgtg catgttaacc ataaagaaat taaaaataaa 3300

ttctaattac acatagcata cagttataag taaaggtgac cattttgctc atccgatttt 3360

gttccctaga gataactact gttaataagt gttgcatgat cagttaaaat tcaaaccaac 3420

aaacactatg ttcaagggat tgtgggtata tacaacaaat atgaacatcc ttttgccttg 3480

cctgcagata ccctcaataa tgctgaaaga cttatacaac attactgctt ccaaagctta 3540

gactatctca ctttgttttc aaaggaggtt ttacgacctt ctaaagagat tgaaattgac 3600

atttcaccta aaactcggga aatgtaaatg acaatattaa ttggtaagag aggaaagaag 3660

aaagaaagaa ggaaggaaag aaagaaagaa ggaaggaagg aaagaaagaa agaaagaaag 3720

aaagagagag aaagaaagaa aaagaaaaaa gagagaaaga gagaaggaaa gaaagagaga 3780

aggaaaggaa aagagaagca aagaaagaga ggagcaaaga aaggaacact tagcactagt 3840

taggagaccc aactctggaa ttatcagcta tatatttaac aaacgttata cttttaaata 3900

gcaaactctt tattgtttca attttatctg gtcaattgga aaaataattt ttgtcttatc 3960

tgtctccttg aaatgtgagg atcaaaggag actaaaacat gatagctttt aaagtctatt 4020

tcagtaaaac agacttatat agaggggttt ttatcatgct ggaacctgga aataaagcaa 4080

accagttaga tgctcagtct ctgccctcac agaattgcag tctgtcctca caaatgtcag 4140

caatagatat gattgccaag cagtgcccca tccagtgctc ttatcccagc tcatcacgat 4200

cttggagttc ccatttctct ctgcaggtgg aactgacctc tgataagaaa agctcctcgg 4260

agaacacatg cctcactatt tgccatctac tttaacaggg ctttgctgca accagactct 4320

ttcaaaagaa gacatgcatt gtgcacaaaa tgaacaagga agtcatgccc tccattcaat 4380

cccttgatgc actggtcaag gaaaagaagg taaaaataaa aggcttttta tttttggtga 4440

ggggagaggt tttacatcct tcagtaaata acgagaagat cacagtcatt ccctcttgac 4500

tacagtatgt tgtagtgtgc agcacaaagg gggaagttat tggtgattgc ctgagggaag 4560

gcaacttctg ccacatcaaa tgctgtggct cacacctacc tctacaaccg ctgagcaaag 4620

cacttgaaac cttgactgtt agaggagcaa agctctggtc acaccaatag gagcctcagt 4680

actttgccaa ggacattttt ctgcaagagt tagttagggt tattagattt agcaaatgaa 4740

aatagaagat atccagttag gtttgaattt taggtaagca gcaggtcttt ttagtataat 4800

atatcctatg caatatttgg gatatactaa aaaaagatcc attgtttatc tgaaattcaa 4860

atgtaactgg gtattgtata ttttgtctgg ccatactaat ccaggtgagt ggaaagaaga 4920

gatccataat gttttaaaat atttgcctga gttcatattc ctataactga taaatgagta 4980

cctttcattg acaaggtaga gaaaataaat aaactgcatt ctcagaagat gattattaca 5040

tagtctaatc caaggaatct atgatgacca aatgaggtcc aagttgcaga ataaattaag 5100

cctcagactt ctgtgtttat gagaagctga ggtttcaaac cagctaaatc ccttaggaca 5160

cttagaaatg ctaagatata cagaataagc tagaaatggc tcttcttcat cttgattatg 5220

gaaaaattta gctgagcaac actcactgtt ggcctcgtat acccctcaag tcaacaaacc 5280

actgggcttg gcattcattc tctcccattc ttcctttcta cctctctttt ccacactcag 5340

cttcagggta agggaccagg aggaccacct cccaagggcc tgatgtactc agtcaaccca 5400

aacaaagtcg atgacctgag caagttcgga aaaaacattg caaacatgtg tcgtgggatt 5460

ccaacataca tggctgagga gatgcaaggt gagtagcatc cctactgtgc accccaagtt 5520

agtgctggtg ggattgtcag actatcctcg cgcgtgtcca tagtgggcac cagtgatgca 5580

gggatggtca tcaaggccaa catttgtgca gtgcttgctc tgtgccaggt actgttctat 5640

gtgctttaag tgtgttaact cggttcttca cagcaatctt ataggttcta ttttaatcct 5700

actttatgga tgaggaaact gaggtacaga gaggtcacaa aatccttgcc tgggtcaatt 5760

ccaagcattt tggctgtgga ttctgtgctc ttaaatatta tggaacactg ccttttaagt 5820

gtgaatcaag agtagactca agtcatattc aaaagaatgc atgaatggct aaatgaaaga 5880

agaatgctaa tagaatctat taactttcta tagctcagac aatcacttaa tttctggaca 5940

ttcaaagaac agctgcacac aaacaaagtg tctacctagg gacctaactt aatggcaatt 6000

ttccagatct ctgaattgat tgatttcatc acaacaagta gataaacctt gacattagca 6060

catagctagt ttggaaaccc ctactccccc aatcccctcc aagaaaagag tccttaaata 6120

gacattaata taggcttctt cttttctctt tattagaggc aagcctgttt ttttactcag 6180

gaacgtgcta cacgaccagt gtactatgga ttgtggacat ttccttctgt ggagacacgg 6240

tggagaacta aacaattttt taaagccact atggatttag tcatctgaat atgctgtgca 6300

gaaaaaatat gggctccagt ggtttttacc atgtcattct gaaatttttc tctactagtt 6360

atgtttgatt tctttaagtt tcaataaaat catttagcat tgaattca 6408

<210> 3
<211> 20
<212> DNA
<213> Artificial Sequence

<220>
<223> E1 sense primer

<400> 3
caacctggtt aagtacaaat 20

<210> 4
<211> 18
<212> DNA
<213> Artificial Sequence

<220>
<223> E1 anti-sense primer

<400> 4
gcagacctcc taaatctc 18

<210> 5

<211>    20
<212>    DNA
<213>    Artificial Sequence

<220>
<223>    E3 sense primer


<400>    5
   caccttttca agtttgctac                                                    20


<210>    6
<211>    20
<212>    DNA
<213>    Artificial Sequence

<220>
<223>    E3 anti-sense primer


<400>    6
attgcacgtt gactacctac                                                   20


<210>    7
<211>    20
<212>    DNA
<213>    Artificial Sequence

<220>
<223>    E4 sense primer


<400>    7
tcactatttg ccatctactt                                                   20


<210>    8
<211>    16
<212>    DNA
<213>    Artificial Sequence

<220>
<223>    E4 anti-sense primer


<400>    8
aaacctctcc cctcac                                                       16


<210>    9
<211>    20
<212>    DNA
<213>    Artificial Sequence

<220>
<223>    E5 sense primer


<400>    9
ctctcccatt cttcctttct                                                   20

<210>    10
<211>    18
<212>    DNA
<213>    Artificial Sequence

<220>
<223>    E5 anti-sense primer


<400>    10
cccaccagca ctaacttg                                                    18


<210>    11
<211>    20
<212>    DNA
<213>    Artificial Sequence

<220>
<223>    E6 sense primer


<400>    11
cattaatata ggcttcttct                                                  20


<210>    12
<211>    18
<212>    DNA
<213>    Artificial Sequence

<220>
<223>    E6 anti-sense primer


<400>    12
cagcatattc agatgact                                                    18


<210>    13
<211>    25
<212>    DNA
<213>    Artificial Sequence

<220>
<223>    methylated sense primer


<400>    13
taatgaatag gagtttatta agcga                                            25


<210>    14
<211>    30
<212>    DNA
<213>    Artificial Sequence

<220>
<223>    methylated anti-sense primer


<400>    14

aaatacataa ataaacataa ctctatcacg                                    30

<210>    15
<211>    30
<212>    DNA
<213>    Artificial Sequence

<220>
<223>    unmethylated sense primer

<400>    15
tttgaataat gaataggagt ttattaagtg                                    30

<210>    16
<211>    30
<212>    DNA
<213>    Artificial Sequence

<220>
<223>    unmethylated anti-sense primer

<400>    16
aatacataaa taaacataac tctatcacac                                    30

<210>    17
<211>    20
<212>    DNA
<213>    Artificial Sequence

<220>
<223>    GAPDH sense primer

<400>    17
acccagaaga ctgtggatgg                                               20

<210>    18
<211>    20
<212>    DNA
<213>    Artificial Sequence

<220>
<223>    GAPDH anti-sense primer

<400>    18
ttctagacgg caggtcaggt                                               20

<210>    19
<211>    20
<212>    DNA
<213>    Artificial Sequence

<220>
<223>    GKN 1 sense primer

<400>    19
caaagtcgat gacctgagca                                                    20

<210>    20
<211>    20
<212>    DNA
<213>    Artificial Sequence

<220>
<223>    GKN 1 anti-sense primer

<400>    20
cttgcctctt gcatctcctc                                                    20

<210>    21
<211>    21
<212>    DNA
<213>    Artificial Sequence

<220>
<223>    GAPDH sense primer

<400>    21
aaatcaagtg gggcgatgct g                                                  21

<210>    22
<211>    21
<212>    DNA
<213>    Artificial Sequence

<220>
<223>    GAPDH anti-sense primer

<400>    22
gcagagatga tgaccctttt g                                                  21

## Description

### [Technical Field]

The preset invention relates to an anticancer composition including a GKN 1, and more particularly, to new use of a GKN 1 gene having excellent activity in suppressing the rate of stomach cancer and metastasis of a cancer cell.

### [Background Art]

The greatest occurrence of stomach cancer is in Korea, Japan, and the like in the world, but the West, such as the United States and Europe has low occurrence of stomach cancer. In Korea, the highest occurrence rate of cancer is stomach cancer, and stomach cancer is the second major cause of death, after lung cancer. Classification of stomach cancer shows that adenocarcinoma occurred gland cells in mucosal membrane of the stomach walls is 95% of the whole cancer, and there are also lymphoma occurred in lymphatic system and gastrointestinal tumor occurred in an interstitial tissue.

It has been confirmed that the most important cause of the outbreak especially is food and drink among various causes of stomach cancer. It is known that a nitrate and nitrite which are plentifully included in the processed meat are powerful carcinogens, and also burnt food and spicy and salty food are the cause of stomach cancer. Beyond that, it has been found that *Helicobacter pylori* that is recently the centre of attention is bacteria causing gastritis and stomach ulcer, and associated with the occurrence of stomach cancer.

In addition, the causes of cancer are as follows: cancer is caused by proliferating cells due to abnormal activation of oncogene causing cancer, or on the contrary by occurring abnormality of tumor inhibitory gene blocking formation of a cancer cell by killing a specific cell through operating apoptosis program or suppressing abnormal proliferation of the cell. Even though cells have a cancer gene that is abnormally activated, when a tumor inhibitory gene expresses a normal activation, the cells do not form cancer and then die. Therefore, in order to be a cancer cell, activation of oncogene as well as inactivation of a tumor inhibitory gene should occur in one cell at the same time.

For this reason, recently, fierce competition in finding targets for treating and using the targets for diagnosing and developing a treatment agent through a function study of genes relating to generation and treatment of cancer all over the world is proceeding actively. Along with activation of a genome study, studies of analyzing human gene DNA chip or proteome are actively proceeding. As the genes relating to cancer are largely found, lists and database relating to many genes are formed. However, specific biological function and cancer relevance about most of the genes in a cell haven't been studied yet or uncertain. Therefore, it is very difficult to use as a gene for diagnosing and targeting and find genes capable of effectively treating cancer. Thus, in addition to a tumor-relating gene that has been found until now, it is necessary to find new genes.

Meanwhile, among the methods for treating cancer now, three main therapeutical methods are a surgical therapy, a drug therapy, and a radiation therapy. As for the drug therapy, the level of suffering is low and a risk of cancer recurrence is relatively low after a treatment as compared with the surgical therapy and radiation therapy. Therefore, there is a rising interest on the drug therapy. For this reason, a number of anticancer drugs are developed and used. Most of the drugs selectively kill the cells that actively divide with the hyperplasia of cancer in mind and thus express anticancer effect. Since the anticancer drug causes severe side effects killing normal cells such as immune cells and hair root cells that are actively divided in a human body, the drugs have a problem in that it is difficult to use for a long period of time. In addition, as a method for treating stomach cancer, there are lymphadenectomy, endoscopic demucosation, and laparoscopic gastrotomy. The endoscopic demucosation is a method of resecting a mucosal membrane having lesion after a lesion part is convexly puffed by injecting saline solution around the gastric mucosal membrane having lesion for early stomach cancer in the mucosal membrane. Therefore, the endoscopic demucosation has an advantage in that it is capable of avoiding the suffering of the stomach resection, but there is a limit in that it can be used only in the case of having low possibility of lymph node metastases among early stomach cancers only on the mucosal membrane.

Accordingly, a discovery of target gene for developing new anticancer drugs capable of preventing and treating cancer in the initial stages using is needed, in which the target gene serves a main function relating to cancer incidence.

### [Disclosure]

### [Technical Problem]

Accordingly, the present inventors researched functions of such a gene from understanding that a GKN 1 gene is expressed at a normal stomach mucosal cell and its expression is reduced at a gastric carcinoma tissue, and as a result, first found the fact that the GKN 1 gene inhibits proliferation and activation of a gastric carcinoma cell, and has a function of inhibiting carcinoma metastasis, and also can be used as a medicine for treating cancer. Thus, the present inventors completed the present invention.

Therefore, an object of the present invention is to provide an anticancer composition including a GKN 1 (Gastrokine 1) protein or polynucleotide encoding the GKN 1 protein.

Another object of the present invention is to provide a method for screening an anticancer drug, including culturing a GKN 1 protein or a recombinant cell expressing the protein and a candidate substance, and measuring effects of the candidate substance on increasing activity of the GKN 1 protein and increasing an intracellular expression level of the GKN 1 protein.

### [Technical Solution]

In order to achieve the above object, an exemplary embodiment of the present invention provides an anticancer composition including a GKN 1 (Gastrokine 1) protein or polynucleotide encoding the GKN 1 protein.

According to an exemplary embodiment of the present invention, the GKN 1 protein may be composed of an amino acid sequence as set forth on SEQ ID NO: 1.

According to an exemplary embodiment of the present invention, the polynucleotide encoding the GKN 1 protein may be composed of a base sequence as set forth on SEQ ID NO: 2.

According to an exemplary embodiment of the present invention, the polynucleotide may be included in an expression vector.

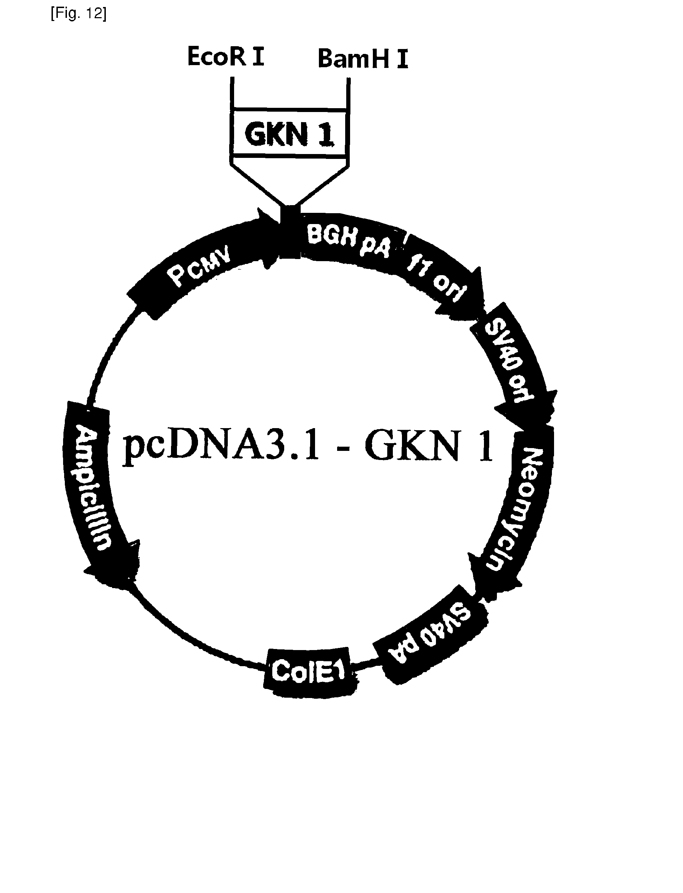

According to an exemplary embodiment of the present invention, the expression vector may be a pcDNA 3.1-GKN 1 having a cleavage map illustrated in FIG. 12.

According to an exemplary embodiment of the present invention, the cancer may be a stomach cancer.

In addition, the present invention provides a method for screening an anticancer drug, in which the method includes culturing a GKN 1 protein or a recombinant cell expressing the protein and a candidate substance; and measuring an effect of the candidate substance on increasing activity of the GKN 1 protein or increasing an intracellular expression level of the GKN 1 protein.

According to an exemplary embodiment of the present invention, when the candidate substance may increase activity of a GKN 1 protein or the expression level of the GKN 1 gene in a cell, the method may further include confirming the candidate substance having anticancer activity as an anticancer drug.

According to an exemplary embodiment of the present invention, the activity and intracellular expression level of the GKN 1 protein may be measured by using any one of method selected from the group consisting of coimmunoprecipitation, a radioimmunoassay (RIA), an enzyme-linked immunosorbent assay (ELISA), an immunohistochemical analysis, a western blotting, and a Fluorescence activated cell sorter (FACS).

According to an exemplary embodiment of the present invention, the anticancer drug may prevent or treat stomach cancer.

### [Effects]

Since the GKN 1 according to the present invention has excellent activity of inhibiting occurrence of a cancer cell and also excellent activity on inhibiting metastatsis of a cancer cell in over-expressing a cancer cell, the GKN 1 may be usefully used as a composition for anticancer therapy.

### [Description of Drawings]

FIG. 1 is results of immune-staining a GKN 1 at a gastric carcinoma tissue, in which (A) shows an immune activity of the GKN 1 at a lacunar cell and a superficial gastric epithelial cell, (B) shows an immunity positive of the GKN 1 at a gastric adenoma, (C) shows a negative immune-staining result at a tubular adenoma, (D) shows an immunity positive at stomach cancer, and (E) and (F) show negative immune-staining results at intestinal type stomach cancer and diffuse type stomach cancer;
FIG. 2 shows a methylation state of a GKN 1, in which (A) shows a methylated DNA (M) and non-methylated DNA (U) as for a GKN 1 gene at a gastric carcinoma tissue (T) and a normal gastric mucosal membrane (N) and (B) shows a methylated DNA (M) and non-methylated DNA (U) as for a GKN 1 gene at a stomach cancer cell line;
FIG. 3 is a graph showing a copy number change of GKN 1 DNA (A) and a fold change of GKN 1 mRNA expression (B) at stomach cancer as compared with a control group;
FIG. 4 shows GKN 1 expressions at intestinal type stomach cancer (I), diffuse type stomach cancer (D), a mixed type stomach cancer (M), and a normal gastric mucosal membrane (N), respectively;
FIG. 5 is (A) a graph showing cell viability at a gastric carcinoma cell transfected with a GKN 1 using a MTT analysis method according to the lapse of time and (B) a graph showing cell proliferation at a gastric carcinoma cell transfected with a GKN 1 using a BrdU analysis method according to the lapse of time;
FIG. 6 shows a result of cell death after GKN 1 transfection according to the lapse of time, measured using an annexin V binding analysis method;
FIG. 7 shows a result of cell death after treating a caspase-3 inhibitor (Z-DEVD-fmk) and caspase-8 inhibitor (Z-IETD-fmk) at 24 hours after GKN 1 transfection, measured using an annexin V binding analysis method;
FIG. 8 shows (A) activities of proteins relating to cell death at 24 hours after GKN 1 transfection, measured by performing a western blotting analysis and (B) results of inhibiting caspase activities after treating a caspase-3 inhibitor (Z-DEVD-fmk) and caspase-8 inhibitor (Z-IETD-fmk), measured by performing a western blotting analysis;
FIG. 9 shows effect of a GKN 1 on cell migration through wound healing method, in which a dotted line indicates limitans of flaw defects;
FIG. 10 shows effects of a GKN 1 on transwell migration at a stomach cancer cell line, AGS, in which (A) is a result of staining a membrane with Diff-Quik and (B) is a graph showing a cell number counted under a light microscopy;
FIG. 11 is a result showing an effect of a GKN 1 on cellular infiltration, measured by performing a matrigel analysis method, in which (A) shows cells infiltrated through a matrix at 24 hours after transfection and (B) is a graph showing the result by counting cell numbers; and
FIG. 12 shows a cleavage map of a pcDNA 3.1-GKN 1 vector as a recombinant vector prepared by inserting a GKN 1 gene according to the present invention.

### [Best Mode]

The present invention provides an anticancer composition including a GKN 1 (Gastrokine 1) as an effective component, and more particularly, an anticancer composition including a GKN 1 (Gastrokine 1) protein or polynucleotide encoding the GKN 1 (Gastrokine 1) protein.

Recently, the present inventors paid attention to a GKN 1 as a new protein isolated from a gastric mucosal cell of a mammal while researching for developing a new cancer treatment drug capable of inhibiting cancer occurrence.

It is known that a GKN 1 (Gastrokine 1) is also referred to as AMP-18 (antrum mucosal protein-18) or CA11, is expressed at gastric antrum, and has activity promoting healing by protecting a mucosal membrane of gastric antrum, and easily allowing injury to be recovered and proliferated after being wounded (Toback. F. G. et al., Am J Physiol Gastrointest Liver Physiol, 285: G344-353, 2003). However, very little is known about other GKN 1 functions as yet.

Accordingly, the present inventors confirmed the fact that the expression of a GKN 1 gene is reduced at a cancer cell unlike a normal cell, and thus speculated that the GKN 1 gene is involved in mechanism of cancer occurrence. Substantially, we first investigated the fact that GKN-1 has anticancer activity.

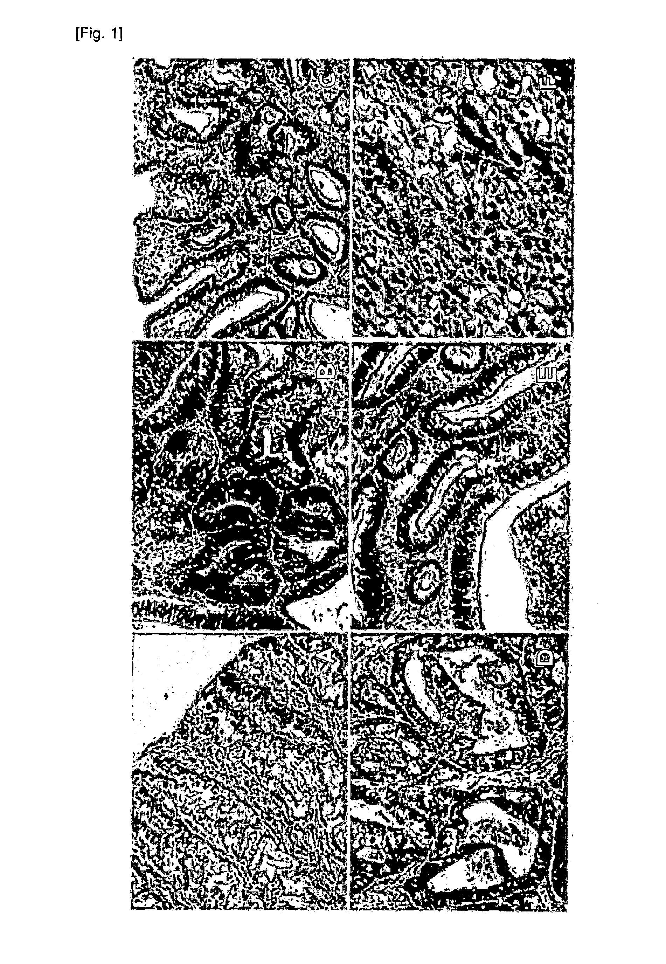

First, according to an exemplary embodiment of the present invention, it can be seen that the expression of the GKN 1 gene is reduced or disappeared at a cancer cell. In other words, according to an exemplary embodiment of the present invention, the expression of a GKN 1 protein is investigated at a gastric carcinoma tissue using an immunohistochemical method as follows: a gastric carcinoma tissue is fixed to prepare a section; by using a GKN 1 antibody and a biotinylated goat anti-mouse antibody, a detection is performed on the section; a slide is cultured with peroxidase-bonded avidin-biotin complex; the slide is counterstained with a Mayer's hematoxylin solution using diaminobenzidine as chromagen; and then the expression is analyzed. As a result, it can be seen that immunity positive is shown in cytoplasm of normal gastric mucosal cell of antrum and corpus and the expression of a GKN 1 is reduced or disappeared at a gastric adenomas and carcinomas tissue (see FIG. 1 and Table 1).

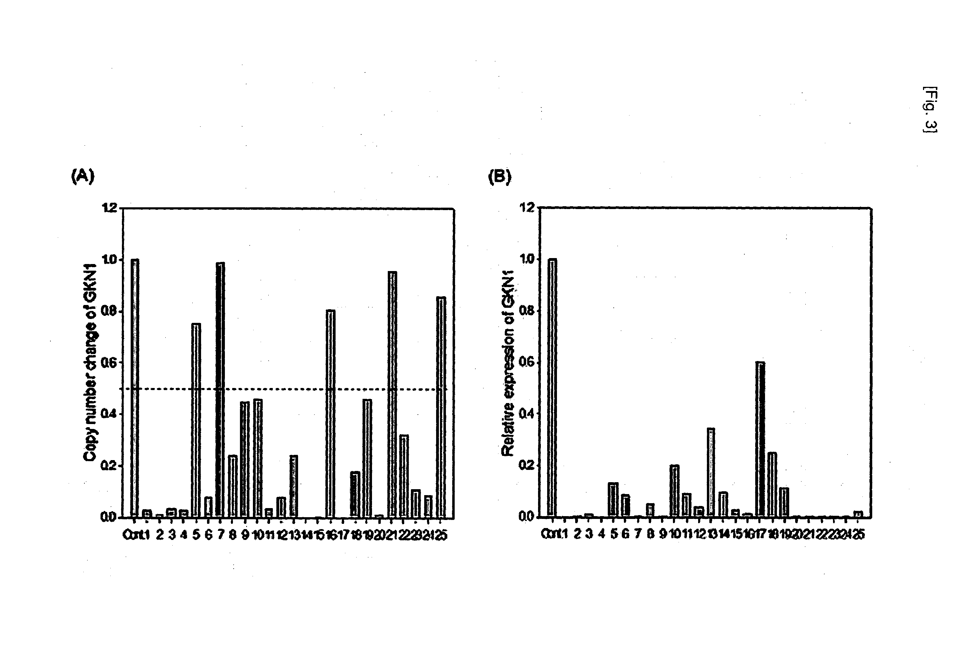

In addition, according to an exemplary embodiment of the present invention, a GKN 1 genome DNA and mRNA extracted from a frozen gastric carcinoma sample were quantified and then subjected to a real time SYBR Green QPCR in a Stratagene Mx 3000P QPCR system. As a result, the copy number of GKN 1 was decreased by at least 50% at DNA of a gastric carcinoma as compared with DNA of a normal gastric mucosal tissue, and as for the expression level of GKN 1 mRNA, GKN 1 gene transcript was expressed at a normal gastric mucosal tissue, while the expression was decreased or disappeared at all of the gastric tissues (see FIG. 3).

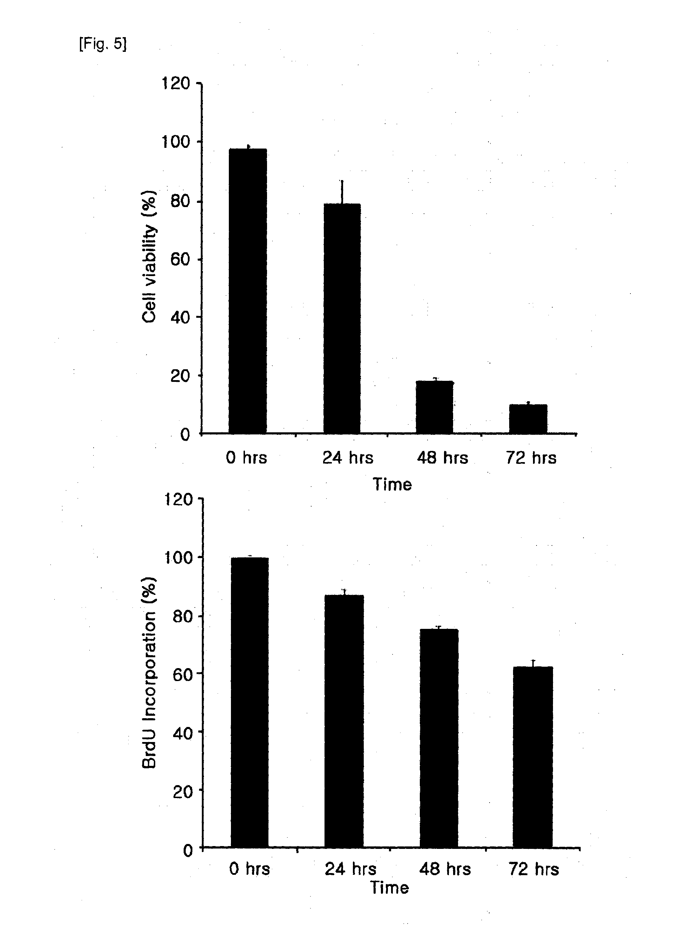

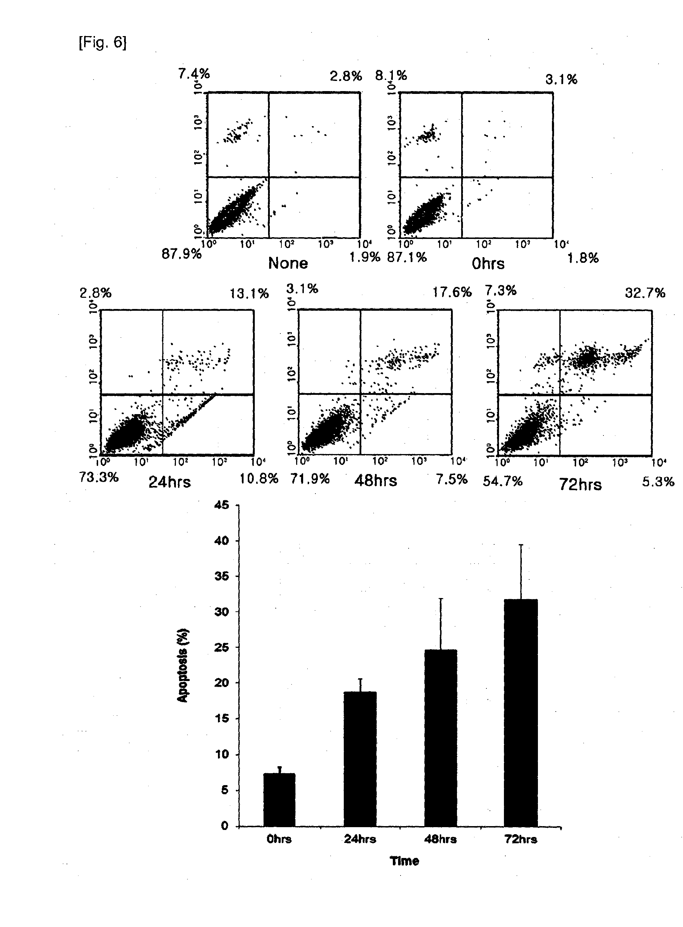

The present inventors predicted on the basis of such a result that the GKN 1 gene according to the present invention may have anticancer activity. Thus, the inventors investigated an effect on cell proliferation and death of cancer cell in the case of over-expressing the GKN 1 gene. In other words, according to an exemplary embodiment of the present invention, the stomach cancer cell line, AGS transfected with the GKN 1 was analyzed by using a MTT analysis and a BrdU bond analysis according to the lapse of time, and as a result, in the transfected cell, cell viability and cell proliferation were remarkably inhibited (see FIG. 5). In addition, as a result of staining FITC-marked annexin V cell, the cell death in the cells transfected with the GKN 1 was increased according to the lapse of time (see FIG. 6).

Accordingly, the GKN 1 according to the present invention inhibits proliferation of a cancer cell and induces death of a cancer cell.

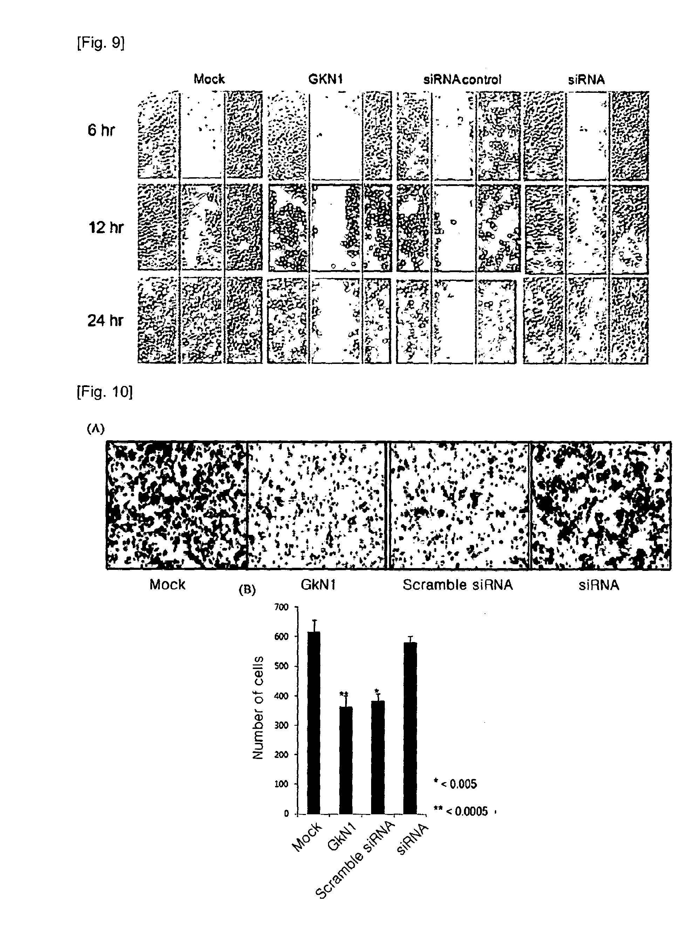

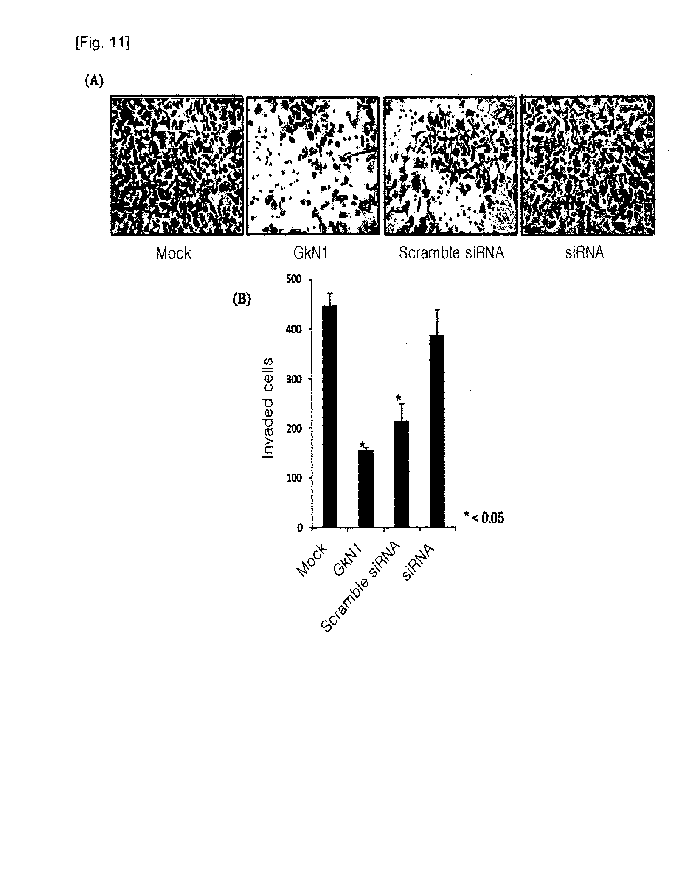

In addition, according to an exemplary embodiment of the present invention, a wound healing, transwell chemotaxis, and infiltration assay were performed in vitro, and as a result, as compared with the AGS cell transfected with the GKN 1 expression vector, the cells transfected with a control expression vector without the GKN 1 gene and the GKN 1 siRNA were remarkably transferred to the wound site after transfection (see FIG. 9), the migration of the AGS cell transfected with the GKN 1 was decreased (see FIG. 10), and the infiltration in the AGS cell, in which the GKN 1 was over-expressed, was remarkably inhibited (see FIG. 11).

Accordingly, as can be seen in the above results, the present inventors can confirm that the GKN 1 inhibits the migration of a cancer cell, resulting on inhibiting conversion of an epithelial cell into an interstitial cell and also inhibiting infiltration in a gastric carcinoma cell line upon over-expressing.

Therefore, the present invention may provide an anticancer composition including a GKN 1 (Gastrokine 1) protein or polynucleotide encoding the GKN 1 (Gastrokine 1) protein.

Preferably, the GKN 1 protein may have an amino acid sequence as set forth on SEQ ID NO: 1 and the polynucleotide encoding the GKN 1 protein may have a base sequence as set forth on SEQ ID NO: 2.

In addition, preferably, the GKN 1 protein according to the present invention may be a functional equivalent to the polypeptide having an amino acid sequence as set forth on SEQ ID NO: 1. Such a "functional equivalent" has sequence homology of at least 60%, preferably 70%, and more preferably 80% or more with an amino acid sequence as set forth on SEQ ID NO: 1 as a result of addition, substitution, or deletion of an amino acid. The functional equivalent is referred to as polypeptide having substantially same activity as the GKN 1 of the present invention. Here, the "substantially same activity" means the activity of GKN 1 as described above. Examples of the functional equivalent may include variants of an amino acid sequence, in which a part of amino acids of the amino acid sequence of the GKN 1 according to the present invention is substituted, deleted, or added. The substitution of amino acid may preferably be conservative substitution, and examples of the conservative substation of the natural amino acid are as follows: aliphatic amino acid (Gly, Ala, Pro), hydrophobic amino acid (Ile, Leu, Val), aromatic amino acid (Phe, Tyr, Trp), acidic amino acid (Asp, Glu), basic amino acid (His, Lys, Arg, Gln, Asn), and sulfur-containing amino acid (Cys, Met). The deletion of amino acid may be preferably located at the part not directly involved in activity of the GKN 1 of the present invention. In addition, the range of the functional equivalent may include a polypeptide derivative in which partial chemical structure of the polypeptide is modified while the basic framework of the GKN 1 and biological activity thereof are maintained. For example, the functional equivalent may include a fusion protein prepared by fusing with other proteins while structural modification and biological activity for changing stability, storability, volatility, solubility, or the like of the polypeptide of the present invention are maintained.

Further, the polynucleotide encoding the GKN 1 (Gastrokine 1) protein may be introduced in an expression vector such as plasmid or a virus vector using known method, and then such an expression vector may be introduced into a target cell as a phenotype by transduction or transfection with various methods that are known in the art.

A method using a plasmid expression vector is a method for transferring a gene, in which the expression vector is approved at FDA as a vector capable of being used for human. The method is a method for transferring a plasmid DNA directly to a human cell (Nabel, E. G. et al., Science, 249:1285-1288, 1990) and the plasmid DNA has an advantage in that it may be homogeneously purified unlike a virus vector. A plasmid expression vector capable of being used for the present invention may include a mammal expression plasmid that is known in the art. According to an exemplary embodiment of the present invention, pcDNA3.1-GKN 1 that is a recombinant expression vector having a cleavage map illustrated in FIG. 12, in which the vector is inserted with a GKN 1 gene, is prepared by using pcDNA3.1 (Invitrogen) plasmid.

The plasmid expression vector including a nucleic acid according to the present invention may be introduced into oncocyte through as a method that is known in the art, that is, transient transfection, microinjection, transduction, cell fusion, calcium phosphate precipitation, liposome-mediated transfection, DEAE Dextran-medicated transfection, polybrene-mediated transfection, electroporation, gene gun, and other known methods for introducing DNA into a cell, but is not limited thereto (Wu. et al., J. Bio. Chem., 267:963-967, 1992; Wu. et al., Bio. Chem., 263:14621-14624, 1988). The transient transfection method may be preferably used.

The vector capable of expressing the GKN 1 may be administrated in a cell, tissue or the body through a known method, for example, local, parenteral, oral, nasal, intravenous, muscular, subcutaneous, or other proper administration. Especially, the vector may be directly injected into a target cancer or oncocyte as an effective amount for treating oncocyte of the target tissue. Especially, in the case of a cancer or tumor present in a body cavity, such as eyes, a gastrointestinal tract, an urogenital organ, lung, and bronchial tubes systems, the pharmaceutical composition of the present invention may be directly injected by using needles, catheters, or other kinds of transferring tubes into a hollow organ that is influenced by a cancer or tumor.

Meanwhile, the GKN 1 of the present invention inhibits proliferation of a cancer cell and promotes cell death, and also has activities of inhibiting metastatsis and infiltration of a cancer cell. Therefore, the present invention may provide a method of screening an anticancer drug, in which the method uses the gene.

In other words, according to the present invention, the method for screening an anticancer drug may include (a) culturing a candidate substance with a GKN 1 protein or a recombinant cell expressing the protein; and (b) measuring an effect of the candidate substance on increasing activity of the GKN 1 protein or increasing a level of the intracellular expression of the GKN 1 protein.

The increase of intracellular level of the GKN 1 protein means that the expression of the GKN 1 gene increases or the concentration of the GKN 1 protein is increased by inhibiting decomposition of the GKN 1 protein. The expression of the GKN 1 gene includes a transcription process of the GKN 1 gene and a translation process into a protein. Therefore, in a case where the candidate substance increases the expression of the GKN 1 gene and the level of the protein in a cell, it may be determined that the candidate substance has anticancer activity.

Furthermore, a method for measuring the activity of the GKN 1 protein and the level of the intracellular GKN 1 protein may include various methods that are known in the art, for example, but is not limited thereto, coimmunoprecipitation, an enzyme-linked immunosorbent assay, a radio immune assay (RIA), an immunohistochemical assay, a western blotting, and a fluorescence activated cell sorter (FACS).

In addition, the screening method of the present invention using GKN 1 as a target may use a high throughput screening (HTS). The HTS is a method for screening for biological activities of many candidate substances simultaneously or almost simultaneously through testing many candidate substances together. As a specific embodiment, the expression level of GKN 1 may be measured by culturing cell lines in a 96-well microtiter plate or 192-well microtiter plate, treating a number of candidate substances to the plate, and then performing an immunochemical method. The wells is typically needed to have a test capacity of 50 µl to 500 µl, and in addition to a plate, many instruments, apparatus, pipettors, robots, a plate washer, and a plate reader may be used commercially to be a 96-well format to be suitable for a broad homogenous system or heterogeneous system test.

Further, the anticancer composition according to the present invention may be used as a pharmaceutical composition capable of preventing and treating a cancer, and the pharmaceutical composition may further include a pharmaceutically acceptable carrier. The "pharmaceutically acceptable composition" means a composition that is physiologically acceptable and does not cause generally gastroenteric trouble, a allergic reaction such as dizziness, or a similar response thereto when administrating to human. Examples of the pharmaceutically acceptable carrier may include carriers for an oral administration, such as lactose, starch, cellulose derivatives, magnesium stearate, and stearic acid, and carriers for a parenteral administration, such as water, suitable oils, saline solutions, aqueous glucose, and glycol, and may further include stabilizer and a preserving agent. The suitable stabilizer may include antioxidant such as sodium bisulfate, sodium sulfite, or ascorbic acid. The suitable preserving agent may include benzakonium chloride, methyl- or propyl-paraben, and chlorobutanol. Other pharmaceutically acceptable carriers disclose in the following document: Remington's Pharmaceutical Sciences, 19th ed., Mack Publishing Company, Easton, PA, 1995. The pharmaceutical composition according to the present invention may be formulated in a suitable type according to a method that is known in the art along with the pharmaceutically acceptable carriers as described above. In other words, the pharmaceutical composition of the present invention may be prepared in types for various parenteral or oral administrations according to the known method, and a representative formulation for a parenteral administration is preferably an isotonic aqueous solution or suspension as a formulation for injection. The formulation for injection may be prepared according to a method that is known in the art using a suitable dispersing agent or wetting agent, and a suspending agent. For example, each component may be dissolved in a saline solution or buffer, and then formulated for injection. In addition, the formulation for an oral administration may include powders, granules, tablets, pills, capsules, and the like, but the present invention is not limited thereto.

The pharmaceutical composition formulated by such a method may be administrated in an effective dose via various routes such as an oral, percutaneous, subcutaneous, or muscular route. The "effective dose" refers to an amount exhibiting effects on preventing or treating when being administrated to a patient. The dose of the pharmaceutical composition according to the present invention may be properly selected according to an administration route, an injection object, age, sex, body weight, difference among individuals, and disease states. Preferably, for the pharmaceutical composition of the present invention, a content of an effective component may be varied according to the degree of disease. It may be repeatedly administrated in an effective dose several times per a day, for example, 1 to 10000 µg/body weight (kg)/day, and more preferably 10 to 1000 mg/body weight (kg)/day. In addition, the anticancer composition of the present invention may be administrated by combining a known compound having an effect on preventing, improving, or treating cancer.

Hereinafter, the present invention will be described in more detail with reference to Examples. It is obvious by a person who skilled in the art that Examples are only for illustrating the present invention in more detail, and the range of the present invention is not limited to Examples.

### <Example 1>

### Preparation of recombinant vector inserted with GKN 1 and transformation thereof into cancer cell

In order to transfect GKN 1 into AGS, a gastric carcinoma cell line, the gastric carcinoma cell line, the AGS was first cultured in a RPMI-1640 medium including 10% fetal bovine serum that was inactivated with heat and 5% CO₂ at 37°C. Then, cDNA of the GKN 1 was amplified by using a PCR method, and an pcDNA3.1-GKN 1 recombinant vector having a cleavage map as illustrated in FIG. 12 was prepared from pcDNA3.1 (Invitrogen, Carlsbad, CA, USA) vector using restriction sites (using EcoRI and BamHI). Then, the recombinant vector was transformated into *Escherchia coli* DH5a aqueous competent cell. The transformated cell was cultured in a LB agar solid medium including ampicillin, and then a culture seed was prepared from the cultured colony to culture in quantity. From the culturing solution, the cells were collected, DNA was purified, and then pcDNA3.1-GKN 1 vector was prepared. Then, the expression vector (total DNA 5 µg) was transfected into the cultured AGS cells using a Lipofectamine Plus Transfection Reagent (Invitrogen) in a dish having a diameter of 60 mm.

### <Example 2>

### Analysis of expression pattern of GKN 1 through immunohistochemical assay

In order for an immunohistochemical assay, a microarray recipient block was designed to have 169 gastric carcinoma tissue samples that were prepared by fixing with a formalin solution and then treating with paraffin. Three tissue cores having a diameter of 2 mm were taken from each of cancers and then transferred into a new recipient paraffin block using a commercially available microarray instrument (Beecher Instruments, Micro-Array Technologies, Silver Spring, MD, USA). Also, a cylinder of a normal gastric mucosal membrane around each of tumors was transferred to the recipient block. All of the samples were cut in a 2 µm section the day before being used and then stored according to a standard protocol. In addition, in order to maximize an immunohistochemical signal, both a method of recovering antigen in a citrate buffer and a method of amplifying the signal with biotinylated tyramide were used. Specifically, the method of recovering epitope induced by heat was as follows: a slide was immersed into a Coplin container filled with 10 mmol/L citrate buffer (pH 6.0); the buffer was boiled in a pressure cooker inside 700W microwave (Nordic Ware, Minneapolis, MN, USA) for 30 minutes; and then the Coplin container was cooled for 20 minutes. Then, using a Renaissance TSA Indirect Kit (NEN Life Science, Boston, A, USA) including streptavidin-peroxidase and biotinylated tyramide, the slide was first washed with a PBS, and then treated in a 1% H2O2 diluted with a PBS at room temperature for 15 minutes in order to remove activity of endogenous peroxidase. Then, the slide was washed with a TNT buffer (0.1 mol/L Tris-HCl, pH 7.4, 0.15 mol/L NaCl, 0.05% Tween 20) for 20 minutes, and then treated with a TNB buffer (0.1 mol/L Tris-HCl, pH 7.4, 0.15 mol/L NaCl, 0.5% blocking reagent). Then, the section was cultured with a GKN 1 antibody (1/100; Sigma-Aldrich Co, St. Louis, MO, USA) at 4°C overnight; detected by using a biotinylated goat anti-mouse antibody (Sigma, St. Louis, MO, USA); and then cultured with a peroxidase-bonded avidin-biotin complex. The section was counterstained with a Mayer hematoxylin solution using diaminobenzidine as chromogen. As for analysis of an antigen staining result, when cytoplasm was positively stained by 30% or more, it was considered positive. Two pathologists were independently tested. As a negative control, non-immunized serum was used instead of a primary antibody.

**[Table 1]**

| Parameters | GKN 1 protein expression | | |
|---|---|---|---|
| | + | - | *P* value |
| **Adenomas** | 4 | 36 | - |
| **Carcinomas** | - | - | - |
| Tumor location | - | - | 0.3722 |
| Upper | 3 | 15 | - |
| Lower | 17 | 155 | - |
| Size | - | - | 0.7814 |
| <6.5 cm | 11 | 99 | - |
| >6.5 cm | 9 | 71 | - |
| No. of metastatic L/N≠ | - | - | 0.3295 |
| N0(=0) | 2 | 19 | - |
| N1(1≤≥6) | 6 | 57 | - |
| N2(7≤≥15) | 10 | 90 | - |
| N3(>15) | 2 | 4 | - |
| Differentiation † | - | - | 0.7459 |
| Intestinal | 9 | 83 | - |
| Diffuse | 11 | 87 | - |
| ≠lymph node; †Lauren's classification | | | |

As a result, as can be seen in FIG. 1, strong immune positive to the GKN 1 was remarkably showed in the middle in a normal gastric mucosal cell cytoplasm of antrum and corpus. However, surrounding corresponding matrix cells such as fibroblast showed negative to the GKN 1.

Further, as shown in the above Table 1, it was observed that the expressions of the GKN 1 were reduced or disappeared in 36 samples (90%) from adenomas and 170 samples (89.5%) from carcinomas, respectively. Furthermore, the expressions of the GKN 1 were observed in 9 (9.7%) samples from 92 intestinal type stomach cancers and 11 (11.2%) samples from 98 diffuse type stomach cancers, respectively. In addition, the expressions of the GKN 1 were reduced and disappeared in 19 (90.5%) samples from early stomach cancer and 151 (95.0%) samples from progressive stomach cancer, respectively.

### <Example 3>

### Analysis of mutation of GKN 1 gene

In order to find what is likely to cause the expression of the GKN 1 decreases in a stomach cancer cell, mutation of the GKN 1 gene was investigated through a base sequence analysis. To achieve this, 81 gastric carcinoma samples and 40 gastric adenomas which were fixed in methacarn were supplied from a hospital, College of Medicine, the Catholic University of Korea, in which 45 gastric carcinoma samples were an intestinal type stomach cancer and 36 samples were a diffuse type stomach cancer. An average size of stomach cancer was 6.5 cm and all of the sizes of gastric ademomas were smaller than 2 cm. All of the samples supplied were not a familial cancer. The genome DNA of a normal mucosal cell that was corresponded to each of cancer cells was amplified with 6-set primers covering the whole coding region of GKN 1 gene, and sequences of the primers are listed in the following Table 2. Each of PCRs (polymerase chain reaction) was performed under a standard condition, and the conditions of the PCR are listed in Table 3. The sequences of PCR products were analyzed by using a cycle sequencing kit (Perkin-Elmer, Foster City, CA, USA).

**[Table 2]**

| 6-set primers sequences | | | |
|---|---|---|---|
| Exon primer | Primer sequence | SEQ ID NO: | Annealing Tm |
| E1 sense | 5'-CAACCTGGTTAAGTACAAAT-3' | 3 | 52°C |
| E1 anti-sense | 5'-GCAGACCTCCTAAATCTC-3' | 4 | |
| E2 sense | 5'-CAACCTGGTTAAGTACAAAT-3' | 3 | 50°C |
| E2 anti-sense | 5'-GCAGACCTCCTAAATCTC-3' | 4 | |
| E3 sense | 5'-CACCTTTTCAAGTTTGCTAC-3' | 5 | 52°C |
| E3 anti-sense | 5'-ATTGCACGTTGACTACCTAC-3' | 6 | |
| E4 sense | 5'-TCACTATTTGCCATCTACTT-3' | 7 | 50°C |
| E4 anti-sense | 5'-AAACCTCTCCCCTCAC-3' | 8 | |
| E5 sense | 5'-CTCTCCCATTCTTCCTTTCT-3' | 9 | 54°C |
| E5 anti-sense | 5'-CCCACCAGCACTAACTTG-3' | 10 | |
| E6 sense | 5'-CATTAATATAGGCTTCTTCT-3' | 11 | 50°C |
| E6 anti-sense | 5'-CAGCATATTCAGATGACT-3' | 12 | |

**[Table 3]**

| PCR conditions | | |
|---|---|---|
| Initially Denatured | 12 min at 94°C | |
| | | ┐ |
| Denatured | 40 sec 94°C | 35 cycle |
| | | ┘ |
| Annealing | 40 sec at 50-54°C | |
| Extension | 40 sec at 72°C | |
| Final Extension | 5 min 72°C | |

As a result, there were no mutations of the GKN 1 gene in gastric adenomas and gastric carcinomas. In order to confirm reliability of the result, the experiments such as a tissue microdissection, PCR, and a base sequence analysis were repeated three times, and thus the results were the same with each other.

Accordingly, it could be found that the decrease of the GKN 1 gene expression in a gastric carcinoma cell was not caused by the mutation.

### <Example 4>

### Analysis of methylation state

Form Example 3, it could be found that the decrease of the GKN 1 gene expression in a gastric carcinoma cell was not caused by the mutation. Thus, in order to analysis whether or not the methylation of a GKN 1 promoter region is involved in the decrease of the GKN 1 gene expression, 25 frozen gastric carcinoma samples and 13 gastric carcinoma cell lines (AGS, MKN-1, MKN-45, MKN-74, KATO-III, SNU-1, SNU-16, SNU-216, SNU-484, SNU-520, SNU-601, SNU-638, SNU-668, SNU-719) were used, in which the samples were supplied from Korea Biobank, Chonnam National University Hwasun Hospital that is a member of National Biobank of Korea that has been funded by Ministry of Health & Welfare. The methylation state in the promoter region of GKN 1 gene was determined by performing a methylation-specific PCR (MSP) after treating sodium bisulfide of DNA. With 5 µl of DNA substituted with bisulfide, MSP was performed by using 2-set primers to a methylated GKN 1 and non-methylated GKN 1. The primer sequences are listed in the following Tables 4 and 5, and a PCR composition and conditions are listed in Tables 6 and 7, respectively. Each of the PCR products was immediately transferred to a 2% agarose gel; then stained with ethidum bromide to be seen under a UV light; and then subjected to an electrophoresis.

As a result, there were no mutations of the GKN 1 gene in gastric adenomas and gastric carcinomas. In order to confirm reliability of the result, the experiments such as a tissue microdissection, PCR, and a base sequence analysis were repeated three times, and thus the results were the same with each other.

Accordingly, it could be found that the decrease of the GKN 1 gene expression in a gastric carcinoma cell was not caused by the mutation.

### <Example 4>

### Analysis of methylation state

Form Example 3, it could be found that the decrease of the GKN 1 gene expression in a gastric carcinoma cell was not caused by the mutation. Thus, in order to analysis whether or not the methylation of a GKN 1 promoter region is involved in the decrease of the GKN 1 gene expression, 25 frozen gastric carcinoma samples and 13 gastric carcinoma cell lines (AGS, MKN-1, MKN-45, MKN-74, KATO-III, SNU-1, SNU-16, SNU-216, SNU-484, SNU-520, SNU-601, SNU-638, SNU-668, SNU-719) were used, in which the samples were supplied from Korea Biobank, Chonnam National University Hwasun Hospital that is a member of National Biobank of Korea that has been funded by Ministry of Health & Welfare. The methylation state in the promoter region of GKN 1 gene was determined by performing a methylation-specific PCR (MSP) after treating sodium bisulfide of DNA. With 5 µl of DNA substituted with bisulfide, MSP was performed by using 2-set primers to a methylated GKN 1 and non-methylated GKN 1. The primer sequences are listed in the following Tables 4 and 5, and a PCR composition and conditions are listed in Tables 6 and 7, respectively. Each of the PCR products was immediately transferred to a 2% agarose gel; then stained with ethidum bromide to be seen under a UV light; and then subjected to an electrophoresis.

**[Table 4]**

| MSP using methylated PCR product | | |
|---|---|---|
| Primer | Primer sequence | SEQ ID NO: |
| methylated sense | 5'-TAATGAATAGGAGTTTATTAAGCGA-3' | 13 |
| methylated anti-sense | 5'-AAATACATAAATAAACATAACTCTATCACG-3' | 14 |

**[Table 5]**

| MSP of non-methylated PCR product | | |
|---|---|---|
| Primer | Primer sequence | SEQ ID NO: |
| unmethylated sense | 5'-TTTGAATAATGAATAGGAGTTTATTAAGTG-3' | 15 |
| unmethylated anti-sense | 5'-AATACATAAATAAACATAACTCTATCACAC-3' | 16 |

**[Table 6]**

| PCR composition | |
|---|---|
| template DNA | 5 µl |
| primer | 0.5 |
| dNTP | 0.2 |
| MgCI2 | 1.5 mM |
| Ampli Taq gold polymerase | 0.4 unit |
| 10X buffer | 3 µl |
| Total | 30 µl |

**[Table 7]**

| PCR condition | | |
|---|---|---|
| Initially Denatured | 1 min at 95°C | |
| Denatured | 30 sec 95°C | ┐ |
| Annealing | 30 sec at 58°C | 40 cycle |
| Extension | 30 sec at 72°C | ┘ |
| Final Extension | 5 min 72°C | |

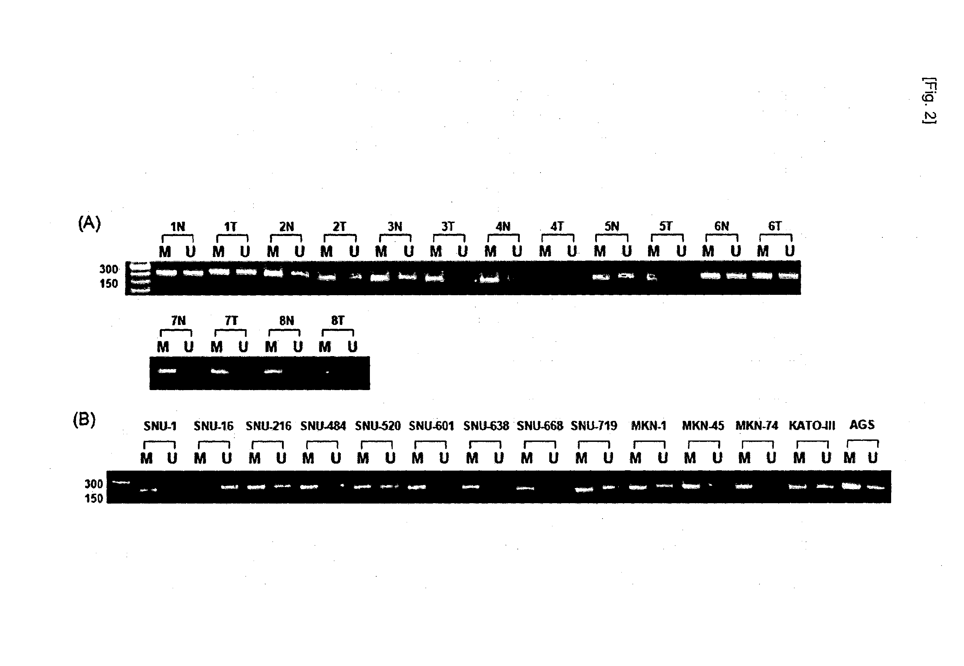

In 25 pairs of noncarcerous gastric mucosal membrane and gastric carcinoma tissues, MSP was performed to analysis bisulfide-modified DNA metylation of the GKN 1 gene. As a result, as illustrated in FIG. 2A, it could be confirmed that both methylated DNA and non-methylated DNA were showed in most cancer tissues and the corresponding normal gastric mucosal membrane. Hypermethylation in the GKN 1 promoter region was observed only in two gastric carcinoma samples (numbers 4 and 8). In addition, as illustrated in FIG. 2B, it could be found that both methylated PCR products and non-methylated PCR products were confirmed in 14 gastric carcinoma cell lines.

Accordingly, it can be confirmed from the above results that the decrease of the GKN 1 gene expression in a gastric carcinoma cell is significantly not involved in the methylation in the GKN 1 gene promoter region.

### <Example 5>

### Analysis of change of DNA copy number of GKN 1 and mRNA expression

Genome DNAs and mRNAs extracted from 25 frozen gastric carcinoma samples were quantified and then subjected to a real time SYBR Green QPCR in a Stratagene Mx 3000P QPCR system. The primers that are specific for detecting the copy number of GKN 1 DNA were designed according to a genome sequence of Genbank, and the primers are listed in the following Table 8. All of the samples were amplified along with oligomernucleotide primers specific for GAPDH gene that is always expressed, and then normalized. The GAPDH primers are listed in the following Table 9.

**[Table 8]**

| Primer | Primer sequence | SEQ ID NO: |
|---|---|---|
| GKN 1 sense | 5'-CAACCTGGTTAAGTACAAAT-3' | 3 |
| GKN 1 anti-sense | 5'-TCACTATTTGCCATCTACTT-3' | 7 |

**[Table 9]**

| Primer | Primer sequence | SEQ ID NO: |
|---|---|---|
| GAPDH sense | 5'-ACCCAGAAGACTGTGGATGG-3' | 17 |
| GAPDH anti-sense | 5'-TTCTAGACGGCAGGTCAGGT-3' | 18 |

The primers for a SYBR Green analysis were deigned on the basis of a nonhomologous DNA sequence specific for a gene, and cDNA for expressing mRNA was synthesized by using a Roche Molecular System reverse transcription kit (Roche, Mannheim, Germany). 50 ng cDNA for a QPCR was amplified by using 20 pmol/µl of each of sense and anti-sense primers and a Fullvelocity SYBR Green QPCR Master Mix (Stratagene, La Jolla, CA, USA) in a Stratagene Mx 3000P QPCR system. A specific oligonucleotide primer for mRNA was prepared according to the information that is known about a GKN 1 gene, and listed in the following Table 10. In order to ensure DNA and mRNA extractions and accuracy of reverse transcription, all of the samples were amplified along with a specific oligonucleotide primer for GAPDH that is always expressed using PCR. The used GAPDH primers are listed in the following Table 11.

**[Table 10]**

| Primer | Primer sequence | SEQ ID NO: |
|---|---|---|
| GKN 1 sense | 5'-CAAAGTCGATGACCTGAGCA-3' | 19 |
| GKN 1 anti-sense | 5'-CTTGCCTCTTGCATCTCCTC-3' | 20 |

**[Table 11]**

| Primer | Primer sequence | SEQ ID NO: |
|---|---|---|
| GAPDH sense | 5'-AAATCAAGTGGGGCGATGCTG-3' | 21 |
| GAPDH anti-sense | 5'-GCAGAGATGATGACCCTTTTG-3' | 22 |

Primers for a SYBR Green analysis were prepared on the basis of a gene-specific non-homologous DNA sequence. A standard curve method was used to quantify a relative amount of a gene expression product, and provided an unit-less normalized expression level capable of being used for a direct comparison of a relative content between a target DNA and mRNA in various samples. All of the samples were cloned for testing, and an average value was used for quantifying.

As a result, as illustrated in FIG. 3A, a copy number of GKN 1 was reduced by 50% or more in 20 stomach cancer DNAs as compared with a surrounding gastric mucosal tissue DNA in a real time-QPCR analysis. In addition, it was detected that in 5 samples of 8 spreading type stomach cancers, 10 samples of 14 intestinal type stomach cancers, and 3 samples of 3 mixed type stomach cancers, copy numbers of the GKN 1 were reduced.

In addition, the expression level of GKN 1 mRNA in a stomach cancer tissue was expressed as a fold change as compared with the control group after normalizing to GAPDH. As a result, as illustrated in FIG. 3B, while all of noncancerous gastric mucosal tissues expressed GKN 1 gene transcript, all of the stomach cancer tissues shown disappeared or reduced expression and 13 stomach cancer cell lines shown complete absence of gene transcript.

### <Example 6>

### Analysis of GKN 1 protein expression in stomach cancer cell line and tissue

As confirmed from Example 2, in order to reconfirm whether or not the level of GKN 1 protein was reduced in a stomach cancer cell line or tissue as compared with a normal cell or tissue, a western blotting method was performed using 25 frozen stomach cancer samples, corresponding surrounding normal gastric tissues, and 13 stomach cancer cell lines as objects. First, the samples were ground into superfine powder under presence of liquid nitrogen using a medicine pestle and a mortar. The ground samples were suspended in a cold Nonidet P-40 solution buffer supplemented with 1X protease inhibitor mix (Roche). Then, cytolysate was isolated by using 10% polyacrylamide gel blotted into a hybond-PVDF transfer membrane (Amersham), probed with an anti-GKN 1 monoclonal antibody (Sigma), and then cultured along with an anti-mouse IgG bonded with a Horse radish peroxicase (HRP). The protein bands were detected by using enhanced chemiluminescence Western blotting detection reagents (Amersham).

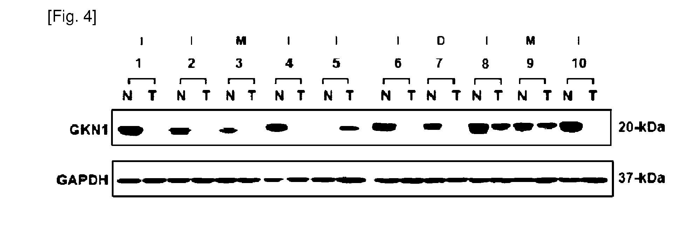

As a result, there were no expressions of GKN 1 gene in any stomach cancer cell lines. In addition, as a result of investigating a stomach cancer tissue by using a western blotting, as illustrated in FIG. 4, the expression of GKN 1 protein was reduced or disappeared by 21 samples (84.0%) among 25 stomach cancer tissues as compared with the corresponding gastric mucosal tissue. In other words, the expressions of GKN 1 protein were reduced in 6 samples (75.0%) of 8 diffuse type stomach cancers, 13 samples (92.8%) of 14 intestinal type stomach cancers, and 2 (66.7%) of 3 mixed type stomach cancers. It was confirmed that the expression of GKN 1 was reduced or disappeared in 2 stomach cancer tissues having hypermethylated gene promoter region.

### <Example 7>

### Analysis of GKN 1 role in cell proliferation and cell death

### <7-1> Cell viability analysis

As described above, from the fact that the expression of GKN 1 gene was reduced in a stomach cancer cell as compared with a normal cell, the present inventors analyzed whether or not the GKN 1 has activity on inhibiting a stomach cancer as the following experiment.

In other words, cell viability was analyzed by using the GKN 1 expression vector prepared in Example 1. The expression vector was transfected into a stomach cancer cell line, and then after 24, 48 and 72 hours, MTT [3-(4,5 dimethylthiazol-2-yl)-2,5-diphenyltetrazoliumbromide] analysis was performed, respectively. Absorbance was measured by using a spectrophotometer at 540 nm and cell viability was measured through a relative expression in a control group. In addition, in order to test an effect of GKN 1 on cell proliferation, the GKN 1 was temporarily transfected into a cell by using a BrdU cell proliferation analysis kit (Millipore, Billerica, MA, USA), and then a BrdU (Bromodeoxyuridine) bonding analysis was performed after 24, 48 and 72 hours, respectively. Absorbance was measured by using a spectrophotometer at 540 nm and cell proliferation was measured through a relative expression in a control group. Here, the non-transfected cells were used as a control group.

As a result, as illustrated in FIG. 5, cell proliferation in the cells transfected with the expression vector expressing GKN 1 was significantly inhibited according the lapse of time as compared with the control group.

### <7-2> Cell death analysis

In order to analysis cell death, an annexin V binding analysis was performed. The annexin V binds with a cell exhibiting phosphatidylserine (PS) at a cell membrane, and propidium iodide (PI) stains a cell DNA having a damaged cell membrane. The transfected AGS cell was washed twice with a cold PBS, and re-suspended in 100 µl binding buffer. For each of samples, 100 µl of supernatant was mixed with 400 µl of a blocking solution. Then, 5 µl of annexin V-FITC and 5 µl of PI (2 µg/ml) were further added into a mixture; the mixture was cultured in a darkroom for 15 minutes; and then cells were analyzed in a fluorescence activated cell sorter (BD Biosciences, USA). The cells that are positive only in fluorescence and are not stained with the PI are considered as death cells. In addition, in order to confirm whether or not the GKN 1 induces apoptosis by caspase, a caspase-3 inhibitor (Z-DEVD-fmk; Calbiochem, San Diego, CA, USA) and caspase-8 inhibitor (Z-IETD-fmk; Calbiochem) for inhibiting cell death were dissolved in dimethylsulphoxide, and then used.

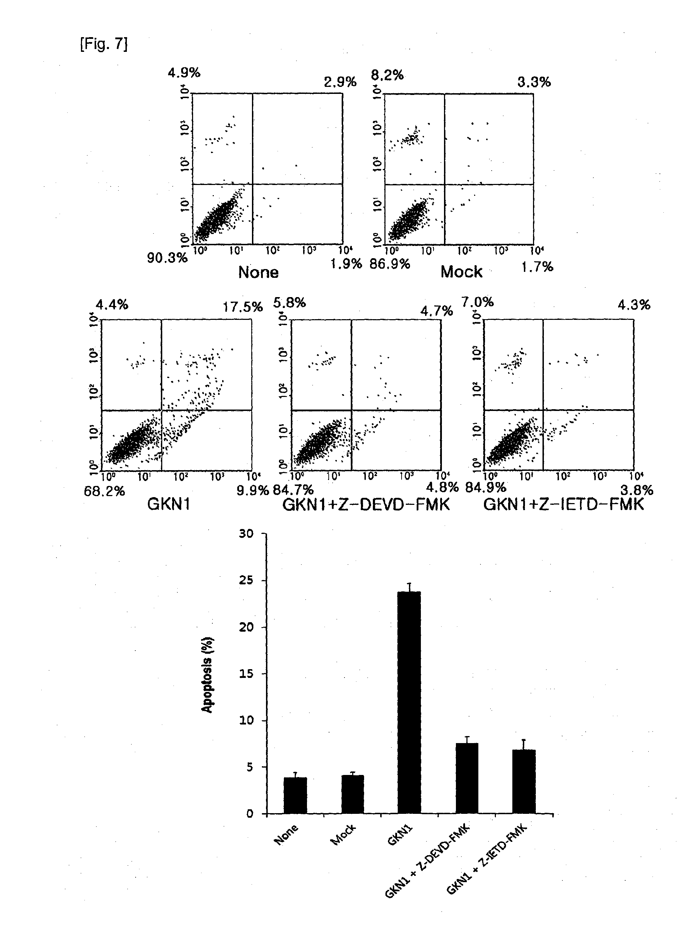

As a result of cell death analysis through a FITC-marked annexin V binding staining, as illustrated in FIG. 6, it could be confirmed that in the stomach cancer cells transfected with the GKN 1 expression vector, the cell death was increased according to the lapse of time. In addition, as a result of treating each of 50 caspase-3 inhibitor (Z-DEVD-fmk) and caspase-8 inhibitor (Z-IETD-fmk) for inhibiting cell death, as illustrated in FIG. 7, it could be confirmed that the cell death caused by the GKN 1 was inhibited by both of caspase inhibitors.

### <7-3> Western blotting analysis of cell death

In order to confirm a molecular mechanism of cell death after transfecting GKN 1, the expression levels of proteins in connection with the cell death were measured. To achieve this, an anti-GKN 1, anti-cleaved caspase 8, anti-BAX, anti-cytochrome c, anti-caspase 3, anti-cleaved caspase 3, anti-PARP, anti-GAPDH, anti-mouse IgG, anti-rabbit IgG, and the like were used; an ECL plus Western blotting detection system (Amersham) was used for detecting a binding antibody; and a band strength after the western blotting was quantified by using a LAS 3000 (Fuji Film, Japan).

Further, in order to confirm whether or not the GKN 1 induces apoptosis due to caspase through a western blotting, a caspase-3 inhibitor and caspase-8 inhibitor for inhibiting cell death were treated, and then western blotting was performed.

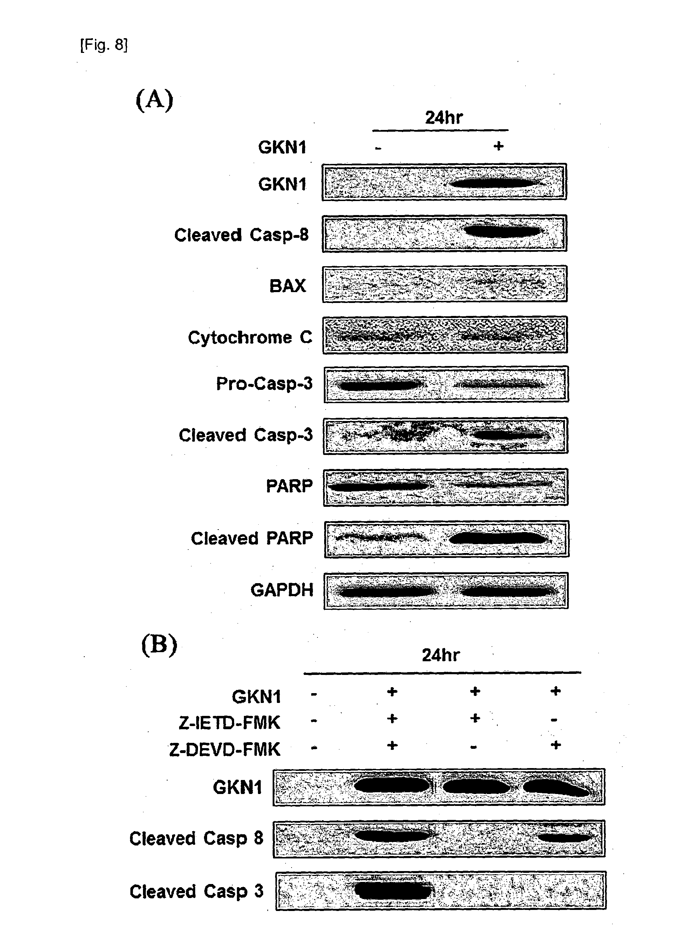

As a result of confirming the proteins in connection with the cell death in the cytolysate transfected with the GKN 1 expression vector through the western blotting analysis, as illustrated in FIG. 8A, it could be confirmed that the cleaved caspase 8, caspase 3, and PARP were detected in the cells transfected with the GKN 1 expression vector. However, the expression changes of proteins in connection with the cell death through a mitochondria route including a BAX and cytochrome c were not detected. In addition, as illustrated in FIG. 8B, the cell death caused by the GKN 1 was definitely inhibited by both caspase inhibitors.

Accordingly, from the above results, the present inventors found the fact that the GKN 1 gene has anticancer activity through promoting cell death of a stomach cancer cell and the cell death is promoted through activity of cell death factors such as caspase-3 and caspase-8.

### <Experimental Example 8>

### Cell migration and infiltration analysis

In order to analysis another anticancer activity of GKN 1, effects of the GKN 1 on cancer cell migration and infiltration were investigated. In other words, each of a GKN 1 expression vector and GKN 1 siRNA was transfected into a AGS cell, and then fusional cell monolayer was injured with a 200 µl pipette tip to prepare a cell-free region. The cell migrations were estimated at 6, 12, and 24 hours after transfecting. Cell motility was analyzed with 8 µm polyvinylpyrrolidine-free polycarbonate (Neuroprobe, Cabin John, MD, USA) coated with gelatin in a 48-well microchemotaxis chamber. Cell suspensions containing 6 × 10⁴ cells were further added to a top chamber of the microchemotaxis chamber and mediums containing 1% bovine serum albumin-RPMI were added to a bottom chamber thereof. Then, the chambers were cultured in a humidification incubator of 5% CO₂ and 37°C for 16 hours. Then, the migrated cells were passed through a membrane and then migrated to a lower chamber, while non-migrated cells were remained in an upper chamber. A cell number was determined by counting the cell number in the lower chamber in a 20x field after repeating the experiment three times. A degree of infiltration was analyzed along with a polycarbonate filter having 8 µm pores coated with 0.5 mm Matrigel in a BD Biocoat MatrigelTM infiltration chamber (BD Bioscience, San Jose, CA, USA). An infiltration chamber having a upper partition containing 1 × 10⁵ cells and the lower chamber containing 5% bovine serum albumin-RPMI medium therein were cultured in a humidification incubator of 5% CO₂ and 37°C for 24 hours. Then, the membrane was fixed and then stained with Diff-Quik. After repeating the experiment twice, the cell number was directly counted in a 20 × field of a microscope.

A wound healing, transwell chemotaxis, and infiltration analysis were performed in vitro. As a result, as illustrated in FIG. 9, while the control expression vector (mock) not inserted with the GKN 1 gene and the cells transfected with GKN 1 siRNA were remarkably migrated into a wound region after 12 hours and 24 hours, respectively as compared with the AGS cells transfected with the GKN 1 expression vector, the migration of the GKN 1-overexpressed cells was inhibited.

Further, the effect of GKN 1 on chemotaxis using serum with a microchemotaxis and chemoattractant was experimented. As a result, as illustrated in FIG. 10, the cell migration of the AGS cells transfected with GKN 1 into a lower chamber was decreased by 41.5% as compared with the cells treated with the control expression vector and AGS cells treated with siRNA.

Furthermore, in order to confirm an effect of GKN 1 on infiltration of AGS stomach cancer cells, the matrigel infiltration was analyzed. As a result, as illustrated in FIG. 11, the infiltration in the GKN 1-overexpressed AGS cell line was remarkably inhibited as compared with the cells transfected with the control expression vector. From above results, it could be confirmed that the over-expression of GKN 1 inhibits infiltration in the AGS cell line, resulting having anticancer activity.

### Statistical analysis

A chi-square test was performed to test a correlation between clinicopathological variables of the GKN 1 expression and stomach cancer. Statistical significance was considered to be a level of less than 0.05 as P value.

The present invention was described with reference to the preferable Examples till now. While the present invention has been shown and described in connection with the exemplary embodiments, it will be apparent to those skilled in the art that modifications and variations can be made without departing from the spirit and scope of the invention as defined by the appended claims.

## Claims

1. An anticancer composition comprising a GKN 1 (Gastrokine 1) protein or polynucleotide encoding a GKN 1 protein.

2. The anticancer composition according to claim 1, wherein the GKN 1 protein consists of an amino acid sequence as set forth on SEQ ID NO: 1.

3. The anticancer composition according to claim 1, wherein the polynucleotide encoding the GKN 1 protein consists of a base sequence as set forth on SEQ ID NO: 2.

4. The anticancer composition according to claim 1, wherein the polynucleotide is included in an expression vector.

5. The anticancer composition according to claim 4, wherein the expression vector is a pcDNA 3.1-GKN 1 having a cleavage map illustrated in FIG. 12.

6. The anticancer composition according to any one of claims 1 to 5, wherein the cancer is stomach cancer.

7. A method for screening an anticancer drug, the method comprising:
culturing a candidate substrate, and a GKN 1 protein or a recombinant cell expressing the GKN 1 protein; and
measuring an effect of the candidate substance on increasing activity of the GKN 1 protein or increasing a level of intracellular expression of the GKN 1 protein.

8. The method according to claim 7, further comprising determining the candidate substance having an anticancer activity as the anticancer drug when the candidate substance allows the activity of the GKN 1 protein to increase or the expression level of the GKN 1 gene in a cell to increase.

9. The method according to claim 8, wherein the activity or intracellular expression level of the GKN 1 protein is measured by using any one of method selected from the group consisting of coimmunoprecipitation, a radioimmunoassay (RIA), an enzyme-linked immunosorbent assay (ELISA), an immunohistochemical analysis, a western blotting, and a Fluorescence activated cell sorter (FACS).

10. The method according to any one of claims 7 to 9, wherein the anticancer drug is capable of preventing or treating stomach cancer.
